# EUROPEAN PATENT APPLICATION

(11) **EP 4 458 814 A1**
(43) Date of publication of application: **06.11.2024**
(21) Application number: 24173869.9
(22) Date of filing: 02.05.2024
(51) Int. Cl.: C07D 251/60, B01J 10/00, F28D 20/00

(54) **METHOD OF REVAMPING OF A PLANT FOR THE PRODUCTION OF MELAMINE, RELATIVE PLANT AND PROCESS WITH ZERO ENVIRONMENTAL IMPACT**

(30) Priority: 03.05.2023 IT 202300008694
(71) Applicant: Proman AG, 8832 Wollerau Schwyz (CH)
(72) Inventor: DE AMICIS, Alberto, I-20097 San Donato Milanese (MI) (IT); DI RUOCCO, Giuseppe, I-23900 Lecco (IT); SANTUCCI, Roberto, I - 21050 Gorla Maggiore (VA) (IT); SASSI, Stefano, 20060 Gessate (MI) (IT)
(74) Representative: De Gregori, Antonella

(57) **Abstract**

The present invention relates to a method for revamping existing plants as well as a plant for the production of melamine with zero environmental impact and the relative process.

## Description

The present invention relates to a method of revamping of a plant for the production of melamine, the relative plant and process with zero environmental impact.

As can be seen daily in every continent on Earth, the planet's climate is changing rapidly, leading to the evolution of increasingly violent and unusual atmospheric phenomena.

According to what has been declared by eminent scientists, the continuous increase in the average annual temperature is the consequence of the variation in the composition of the atmosphere and this change is especially due to the continuous increase in the quantities of greenhouse gases which, as the name suggests, cause a greenhouse effect, heating the surface of the planet and in particular increasing the temperature of the oceans. This heating of the oceans leads to an increase in evaporation and the diversion of currents at high altitudes, with consequent periods of drought and sudden floods with increasingly violent and catastrophic winds.

One of the gases that contributes most to the greenhouse effect is certainly carbon dioxide and it is therefore a priority to reduce as much as possible any source of production of this gas.

As is known, carbon dioxide is formed following the combustion of materials containing carbon such as for example fossil fuels which, in addition to the fact that they are not renewable and therefore destined to slow but inevitable exhaustion, constitute, with their combustion, the major source of air pollution.

Reducing the use of fossil fuels has therefore become an absolute priority and attempts are in fact being made to replace them, as far as possible, with renewable energy sources, or at least with mixed energy sources, i.e. produced with the use of renewable and fossil energy, such as, for example, using electric energy to replace sources of heat from the direct combustion of coal, gas or petroleum derivatives.

It is in fact known that electric energy can be produced by plants with steam turbines, i.e. obtained from the combustion of fossil fuels such as coal, gas or petroleum derivatives, but it can also be obtained from other sources, mainly hydroelectric power plants, photovoltaic systems, wind power systems or nuclear power plants; consequently the replacement of fossil fuel furnaces with electric heating systems represents a clear improvement compared to the current situation.

For years, energy-intensive industries have preferred, when possible, using fossil fuels as a direct source of heating, given the lower cost of these fuels compared to the use of electric energy, thus obtaining a greater profit, but at the same time aggravating the situation of air pollution both locally and globally.

In recent years this senseless way of proceeding has become evident and both the political and industrial world is starting to look for alternatives in order to reduce as much as possible the sources of pollution scattered throughout the territory, as they are more difficult to control and transform into industrial processes with a low ecological impact, thus giving preference to centralized sources of energy production, more easily controllable and distributable in the area: among these the most important is certainly electric energy which can be produced with renewable sources.

In plants destined for the production of melamine, the use of energy is high and is mainly concentrated in supplying the heat necessary for the transformation reaction of urea into melamine which, being endothermic as a whole, needs a continuous supply of heat. The transformation of urea into melamine takes place according to the following overall reaction scheme:

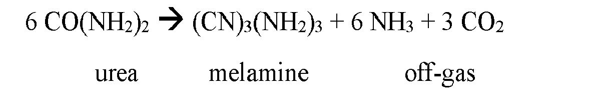

This heat is currently supplied by the circulation of a mixture of salts, normally called molten salts, with a high melting point, which are then heated in turn by specific furnaces which operate by combustion of fuel gas or liquid petroleum derivatives.

The Applicant, a world leader in the supply of plants and technology for the production of melamine, has worked and continues to work to improve the environmental impact of its technology, completely eliminating any liquid or solid process discharge and the emission of carbon dioxide into the atmosphere, with the only exception of carbon dioxide produced by the combustion necessary for supplying the reaction heat to the reactor.

The carbon dioxide that is produced as off-gas from the transformation reaction of urea into melamine is, on the other hand, already recycled to the urea production plant and is not discharged into the atmosphere.

The Applicant therefore decided to explore the topic further and increase the activity aimed at obtaining a complete energy transition, setting itself the objective of obtaining a plant and a process for the production of melamine which operate according to criteria of full environmental sustainability.

In the melamine production process, it should be pointed out that the design of the reactor is certainly one of the most important factors in obtaining a high conversion yield of urea into melamine and therefore any modification of the internal configuration of the reactor, i.e. of the process side of the reactor, the area where the reactions which lead to the formation of melamine take place, should be avoided, or at least reduced as much as possible, in view of the very high yields already achieved by the Applicant's melamine production process.

In the case of already existing melamine production plants, the objective of the present invention can be achieved by revamping the plant itself with the objective to provide a plant, and the relative process, for the production of melamine with zero environmental impact with reference to all solid, liquid and gaseous emissions, while maintaining very high transformation yields of urea into melamine.

The scope of the following invention is therefore also to identify a reactor and a process which operate with the total elimination of both any carbon dioxide emission into the atmosphere and any liquid or solid discharge.

The present invention therefore relates to a method for revamping a plant for the production of melamine which comprises replacing a heat supply system to the reactor already present in the plant with a heat supply system to the reactor suitable for using energy from renewable or partially renewable sources, said plant not producing any emission of CO₂ into the atmosphere following the supply of heat to the reactor.

The various alternative solutions for supplying heat to the reactor in an environmentally friendly way, i.e. with a system for supplying heat to the reactor suitable for using energy from renewable or partially renewable sources indicated above, respectively comprise:
- an electrically heated furnace capable of supplying the necessary heat to the circulation system of molten salts, keeping the configuration of the reactor unchanged and the molten salts continuing to circulate inside the reactor, providing the necessary heat; in the case of the revamping of a plant, the electric heating furnace replaces the combustion furnace which is eliminated; or
- a heat exchanger suitable for supplying the necessary heat to the molten salt circulation system, and a heat recovery system, direct or indirect, from renewable or partially renewable sources, preferably powered by electric or solar energy, keeping the configuration of the reactor unchanged and the molten salts continuing to circulate inside the reactor, providing the necessary heat; in the case of the revamping of a plant, the heat exchanger and the heat recovery system replace the combustion furnace which is eliminated; or
- an electric heating system directly applied to the reactor, thus completely modifying the heat supply system to the reactor; in the case of the revamping of a plant, therefore, the heating system of the reactor is eliminated through the circulation of the molten salts consisting of the combustion furnace and the system for the circulation of the molten salts through the reactor, consisting of a tank - pumps - internal and external pipes.

The method for revamping a plant for the production of melamine which comprises the replacement of the heat supply system to the reactor already present in the plant with the heat supply system to the reactor according to the present invention, i.e. with a heat supply to the reactor suitable for using energy from renewable or partially renewable sources indicated above, therefore preferably comprises the following steps:
- the combustion furnace, suitable for supplying the necessary heat to the circulation system of molten salts, and relative accessory elements, is replaced by an electrically heated furnace, keeping the configuration of the reactor and the molten salt circulation system of the plant unchanged; or
- the combustion furnace, suitable for supplying the necessary heat to the circulation system of molten salts, and relative accessory elements, is replaced by a heat exchanger suitable for supplying the necessary heat to the circulation system of molten salts, connected to a heat recovery system, direct or indirect, from renewable or partially renewable sources, preferably powered by electric or solar energy, keeping the configuration of the reactor and the molten salt circulation system of the plant unchanged; or
- the combustion furnace, suitable for supplying the necessary heat to the circulation system of molten salts, the relative accessory elements, and the system suitable for circulating the molten salts through the reactor, consisting of a tank - pumps - internal and external pipes, are replaced by an electric heating system applied directly to the reactor.

Thus in the above case of revamping an already existing plant, all the alternative solutions indicated above involve eliminating the combustion furnace and, in the third alternative, the molten salt circulation system is also eliminated.

The alternatives indicated above have surprisingly proved to be particularly efficient in replacing heat supply systems to the reactor based on combustion heating systems present in a plant for the production of melamine with an environmentally friendly system such as a heat supply system to the reactor capable of using energy from renewable or partially renewable sources, preferably electric energy.

The present invention relates also to a new plant for the production of melamine characterized in that the heat supply system to a melamine synthesis reactor is a supply system suitable for using energy from renewable or partially renewable sources which comprises:
- an electrically heated furnace suitable for supplying heat to a circulation system of molten salts and a circulation system of molten salts; or
- a heat exchanger suitable for supplying heat to a circulation system of molten salts, connected to a direct or indirect heat recovery system from renewable or partially renewable sources, preferably powered by electric or solar energy, and a circulation system of molten salts; or
- an electric heating system applied directly to the reactor,
said plant not producing any emission of CO₂ into the atmosphere following the supply of heat to the reactor.

The present invention further relates also to the relative process for the production of melamine operating at high pressure, i.e. without the use of a catalyst, characterized in that, in the synthesis step of melamine from urea, the heat to the reactor is supplied, directly or indirectly, through a renewable or partially renewable energy source, preferably electric or solar energy, more preferably electric energy, said process being preferably carried out in the plant according to the present invention.

The first advantage therefore of the solution according to the present invention is that there is no emission of CO₂ into the atmosphere following the supply of heat to the reactor.

The plant according to the present invention, wherein the heat supply system to the reactor is the electric heating system directly applied to the reactor, preferably comprises:
- a plurality of tubes 10 inside which electric heating elements 9 are housed, wherein the external side of the tubes 10 is in contact with the process fluids to which it supplies the necessary reaction heat;
- a plurality of heating elements 9 powered by electric energy 8, situated inside the tubes 10;
- a central internal circulation tube 6 suitable for guaranteeing the internal circulation of the process fluids;
- one or more inlets (F) of raw materials and one or more outlets (G) of the reaction products.

The first embodiment of the plant according to the invention thus consists, with respect to the state of the art, in replacing the combustion furnace, which uses fossil fuels for heating the molten salts, with a furnace for heating the molten salts which uses a renewable energy source, preferably electric energy, maintaining their circulation inside the reactor. In this embodiment, therefore, the system suitable for the circulation of the molten salts through the reactor is still present, consisting of a tank - pumps - internal and external pipes, which does not undergo variations.

The second embodiment of the plant according to the invention consists, with respect to the state of the art, in replacing the combustion furnace which uses fossil fuels for heating the molten salts, and the relative accessory elements, with a heat exchanger which supplies heat to the molten salts, recovering this heat from other renewable or partially renewable sources, such as for example electric heating systems or solar heating systems (Thermal Energy Storage TES), maintaining the circulation of the molten salts inside the reactor. Also in this embodiment, therefore, the system suitable for the circulation of molten salts through the reactor is present, consisting of a tank - pumps - internal and external pipes, which does not undergo variations.

The third embodiment of the plant according to the invention consists, with respect to the state of the art, in a complete elimination of the entire molten salt system: therefore, in addition to the combustion furnace which uses fossil fuels for heating the molten salts, and the relative accessory elements, the system suitable for the circulation of the molten salts through the reactor, consisting of a tank - pumps - internal and external pipes has been eliminated, creating an electric heating system which supplies the reaction heat directly to the reactor without the use of intermediate means, such as molten salts.

This third embodiment of the plant according to the invention results in a greater energy efficiency with respect to the other embodiments as the transfer of energy from the form of electric energy to the form of thermal energy takes place directly without passing through a thermal transfer fluid (molten salts), with a consequent saving in heat losses linked to the presence of tanks-pumps-pipes that form the molten salt system.

The third embodiment is certainly preferable as it also allows for an important plant-engineering simplification. The insertion of an electric heating system in the reactor, in fact, allows the complete elimination of the entire heating system of the reactor through the circulation of molten salts, necessary according to the state of the art, which comprises the fossil fuel heating furnace with relative auxiliary equipment such as the combustion air fan and pre-heater, the fume circulator and the entire molten salt circulation system, consisting of circulation pumps, tanks and relative pipes.

Furthermore, in consideration of the high number of plants for the production of melamine already operating or under construction, the replacement of the existing reaction heat supply systems to the reactor with the heat supply system to the reactor suitable for using energy from renewable or partially renewable sources according to the present invention, is easily applicable, thus also making already existing plants eco-sustainable, and eliminating the source of CO₂ emission into the atmosphere, due to the reaction heat supply system to the reactor.

This application, consisting of a revamping of the plant, leads, in fact, to an upgrading of the plant to which it is applied.

Accessory elements of the furnace refer to fans, heat exchangers, flow-regulation valves, gas-pressure reduction and control system, air/gas ratio regulation system, temperature control and safety, burner control and safety system, burner start-up safety and control system, etc.

As mentioned above, in the melamine production process according to the present invention, in the synthesis step of melamine from urea, the heat to the reactor is supplied directly or indirectly, through a renewable or partially renewable energy source, preferably electric or solar energy, more preferably electric energy.

Furthermore, in the melamine production process according to the present invention the purification of melamine is effected through the use of ammonia, wherein ammonia is produced by the transformation reaction of urea into melamine: it is therefore ammonia already present in the process, which is then fully recovered and re-used in the process.

A further advantage of the process therefore lies in the fact that it does not require the use of chemicals not deriving from the reaction itself, and which, by reacting with other compounds of the process, would inevitably give rise to by-products which would then have to be continuously eliminated from the process to avoid accumulation which would compromise the quality of the final melamine.

The use of chemicals unrelated to the reaction itself would also require a continuous discharge of mother liquor which, in addition to containing the compounds deriving from the chemical used, for example compounds such as sodium carbonates and sodium salts of reaction by-products deriving from the use of caustic soda for maintaining the correct pH, would also contain a certain percentage of dissolved melamine due to its solubility, resulting in a decrease in the overall process yield.

Further characteristics and advantages of the present invention will become evident from the following detailed description of some of its preferred embodiments, made with reference to the attached figures.

The various features in the individual configurations can be combined with each other as desired according to the previous description, should use be made of the benefits specifically resulting from a particular combination.
In said figures,
- figure 1 is a schematic block representation of a plant/process according to the state of the art;
- figure 2 is a schematic block representation of a first embodiment of the present invention;
- figure 3 is a schematic block representation of a second embodiment of the present invention;
- figure 4 is a schematic block representation of a third embodiment of the present invention;
- figure 4A is a schematic representation of the reactor according to the state of the art;
- figure 4B is a detailed representation of the reactor of figure 4.

In the following description, for the illustration of the figures, identical reference numbers are used for indicating construction elements with the same function. Furthermore, for clarity of illustration, some reference numbers may not be repeated in all the figures.

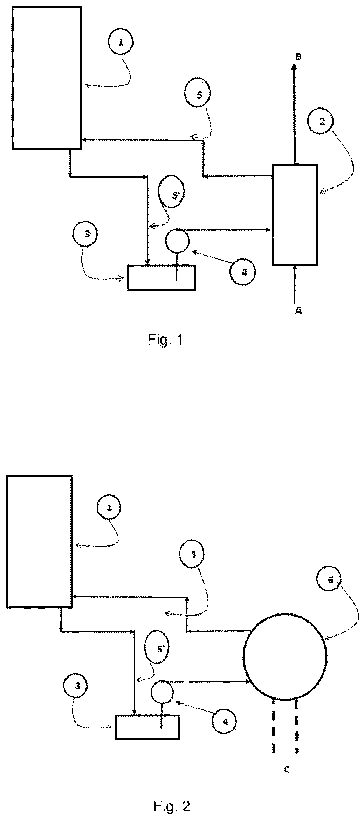

Figure 1, representative of the state of the art, shows the melamine synthesis reactor 1, of which the process part is not shown in the figure as it is not subject to variations, which is brought to and kept at the right temperature by a stream of molten salts, heated by the fossil combustion furnace 2, said stream circulates through a system of pipes 5, 5', pushed by the pump 4 which collects the molten salts from the tank 3, where said stream 5' of molten salts returns after supplying the reactor with the heat necessary for the reaction. The fuel is fed from line A to the fossil combustion furnace 2, whereas from line B, the combustion gases of the furnace are released into the atmosphere.

From figure 1 it is evident that all the combustion gases of the coal furnace, from which heat can be and is recovered on the basis of the construction technology of the furnace, are then discharged into the atmosphere. As these gases mainly consist of an excess of combustion air, CO₂ and water (final compounds of a fossil combustion) it is evident that they are currently one of the main sources of atmospheric pollution of a melamine plant with particular reference to CO₂.

As previously indicated, the Applicant's melamine production technology has already eliminated any polluting discharge from the plant, at the same time reducing all energy consumption through heat recovery, and not requiring the addition of chemicals extraneous to the reaction for the production of melamine, whose use would then require the elimination of the compounds deriving from the same, it does not require any purge either solid or liquid; the elimination of CO₂ discharge into the atmosphere is therefore of primary importance, thus creating a completely and totally GREEN technology, i.e. with zero environmental impact.

Figure 2 represents a first embodiment of the present invention in which the fossil combustion furnace is replaced by an electric furnace 6. This system uses the same circuit and the same equipment as Figure 1 with the difference that the furnace is an electric furnace, powered by electric energy C, in which the molten salts are heated specifically through the use of electric energy. In accordance with this embodiment, it is not necessary to apply any modifications to the production reactor and to the supply system (or circuit) of the molten salts. This solution eliminates the use of fossil fuels and, consequently, the discharge of CO₂ into the atmosphere. This embodiment is particularly advantageous for the revamping of existing plants, whatever technology may be used, thus carrying out a revamping aimed at eliminating the emission of CO₂ into the atmosphere. In the particular case in which the revamping is carried out on a plant operating according to the Applicant's latest generation technology, this would make the plant totally GREEN, i.e. with zero environmental impact.

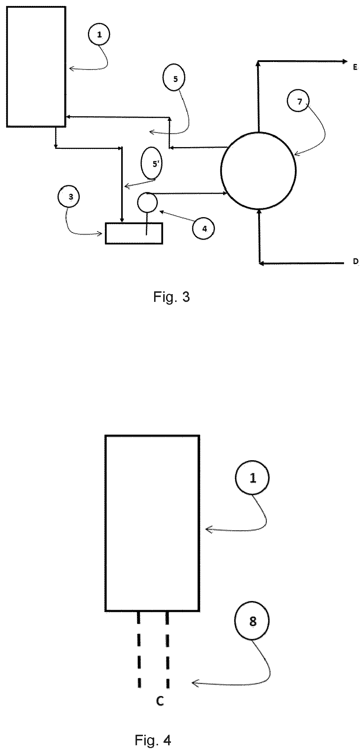

Figure 3 represents a second embodiment of the present invention, in which the fossil fuel furnace is replaced by a heat exchanger 7 which uses, as heating means, the salts used for accumulating heat from solar heating systems (TES or thermal energy storage) not shown in the figure. The heated salts from the TES systems are fed from D and the molten salts are sent to TES from E. This embodiment of the present invention is particularly interesting when the melamine system is located, or is constructed, in the vicinity of a solar heating system.

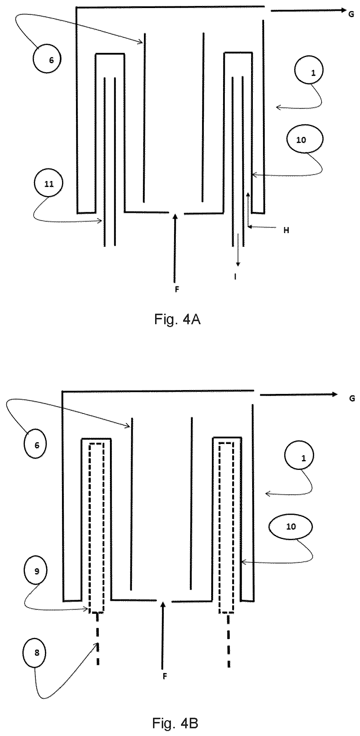

Figure 4 represents a third embodiment in which the electric heating system C is applied directly to the melamine synthesis reactor 1, allowing the elimination of the entire molten salt supply system. The fossil combustion furnace, the tank, the circulation pumps, the pipes and all the relative accessories are therefore eliminated; this system is also applicable to existing plants that use high-pressure technology without changing the internal configuration of the reactor and thus ensuring maintenance of the reaction yields.

For a better understanding the third embodiment and the differences following a revamping process of an already existing plant, figures 4A and 4B show the difference between the reactor 1 before and after the application of the electric heating system to the reactor: in particular, figure 4A shows the reactor 1 before the application of the electric heating system, which therefore provides the heat supply system to the reactor according to the state of the art and figure 4B shows the reactor 1 after the application of the electric heating system to the reactor with the elimination of the entire supply system of molten salts.

Figure 4A represents the internal configuration of the reactor 1 heated with molten salts, whereas Figure 4B represents the internal configuration of the reactor 1 heated by means of electric energy according to one of the embodiments of the present invention.

In particular, in figure 4A the internal process side of reactor 1 is mainly composed of:
- a plurality of tubes 10 inside which the molten salts circulate (entering from H and exiting from I), wherein the outer side of each tube 10 is in contact with the process fluids to which it supplies the necessary reaction heat;
- a plurality of tubes 11, inside the tubes 10, from which the molten salts exit after having supplied heat to the process fluids;
- a central internal circulation tube 6 suitable for guaranteeing the internal circulation of the process fluids;
- one or more inlets F of raw materials and one or more outlets G of the reaction products.

In figure 4B the internal process side of the reactor is mainly composed of:
- a plurality of tubes 10 inside which the electric heating elements 9 are housed, whereas the outer side of the tubes 10 is in contact with the process fluids to which it supplies the necessary reaction heat;
- a plurality of heating elements 9 powered by electric energy 8, located inside the tubes 10;
- a central internal circulation tube 6 suitable for guaranteeing the internal circulation of the process fluids;
- one or more inlets F of raw materials and one or more outlets G of the reaction products.

As can be seen by comparing figures 4A and 4B, the heating elements 9 are positioned in place of the molten salt outlet tubes 11, without it being necessary therefore to apply any modification to the process side of the reactor in the case of a plant revamping, thus guaranteeing the maintenance of previous performances, especially in terms of extremely high yields.

A further possible variant of the solution according to the present invention, not shown in the figures, consists in exploiting the Joule effect, supplying the reaction heat to the reactor by means of an electric power supply system wherein the internal tubes 10 of the reactor 1 are directly used as heating elements. In this case the power supply takes place directly at the two ends of each single tube 10.

From the above description, the features of the system for supplying heat to the reactor for melamine production plants are evident as also the relative melamine production process object of the present invention, together with the relative advantages.

From the embodiments described above, further variants are possible, without departing from the teaching of the invention.

Finally, it is evident that a system for supplying heat to the reactor for melamine production plants and a melamine production plant thus conceived can undergo numerous modifications and variations, all falling within the scope of the invention; furthermore, all the details can be replaced by technically equivalent elements. In practice, the materials used, as also the dimensions, can vary according to technical requirements
As previously indicated, the first advantage of the solution according to the present invention is that there is no emission of CO₂ into the atmosphere following the supply of heat to the reactor.

Furthermore, the third embodiment of the invention is particularly advantageous as it ensures greater energy efficiency even with respect to the other embodiments as the transfer of energy from the form of electric energy to the form of thermal energy takes place directly without passing through a thermal transfer fluid (molten salts), with a consequent saving of thermal dispersions linked to the presence of tanks-pumps-pipes that form the molten salt system. In this third embodiment, in fact, there is an overall efficiency of the electric energy furnace equal to 100% (against an overall efficiency of the furnace of 85% in the other embodiments) with a further advantage of a 15% saving of electric energy.

Furthermore, this embodiment also allows an important simplification of the plant engineering and the construction of the reactor is much simpler, the need for interrupting the activity of the reactor, in the event of a break-down of the furnace, is also reduced.

From the point of view of a plant-engineering simplification, reference should also be made to the complexity and amount of equipment and accessories that make up a fossil combustion furnace. It should in fact be remembered that the term "furnace" also comprises various accessory elements or equipment, in addition to the actual furnace in which the heat exchange between combusted gas and molten salts takes place, of which the main elements are: fans, heat exchangers, flow-regulation valves, gas-pressure reduction and control system, air/gas ratio regulation system, temperature control and safety, burner control and safety system, burner start-up safety and control system, etc. To these elements or pieces of equipment, those relating to the molten salt circulation system should be added, which mainly comprise the circulation pumps, the control valves, the inertization control of the circuit using nitrogen, the temperature detectors of the safety system.

From what is listed above, it can be easily understood how the whole system is subject to shutdowns in the event of a malfunctioning of even only one of the individual elements/pieces of equipment that form the complex system called the "furnace" in its entirety and whose elimination represents a considerable plant simplification especially in the case of the third embodiment of the present invention which provides for a complete replacement of this system with an electric heating system applied directly to the reactor.

The third embodiment of the present invention also corresponds to a constructive simplification of the reactor due to the fact that, as the whole internal distribution and circulation system of the molten salts is not present, all of the tubes, positioned inside the process tubes, which constitute the circulation, collection and outlet system of the molten salts from the reactor, are no longer necessary. These internal tubes are necessarily all connected by means of a manifold and must therefore be installed and/or uninstalled jointly, all together, and this is obviously not an easy operation; in the case of electric heating, the individual elements can however be simply installed and removed individually, and therefore replaced individually in the event of malfunctioning, without jeopardizing the continuous operation of the reactor.

Other features and advantages of the invention will become evident from the following example provided for illustrative and non-limiting purposes.

### EXAMPLE

An 80,000 MTPY melamine plant, based on 8,000 h/y of operation, has an hourly production of 10 T/h of melamine produced, which corresponds to an hourly consumption of methane, for supplying the heat necessary for the reaction, equal to 1,013 kg/h (equal to 101.3 kg/ton).

This methane consumption value must be increased in consideration of the efficiency of the furnace, the thermal dispersions of the molten salt circuit and the conditions of the combustion fumes which are discharged into the atmosphere; in consideration of these factors, and proven by practical consumption data, it has been ascertained that the total efficiency of the system is equal to 85% therefore the actual consumption of methane is 1,192 Kg/h equal to 14,280,160 Kcal/h (1,192 Kg /h X 11,980 Kcal/Kg).

Assuming a complete combustion, this amount of methane generates 3,278 kg/h of carbon dioxide which is discharged into the atmosphere, corresponding to an environmental impact of 26,224 tons/year (3,278 X 8,000).

All the embodiments of the present invention, on the contrary, characterized by supplying the reaction heat to the reactor by means of electric energy, guarantee the complete zeroing of the carbon dioxide discharge into the atmosphere.

As already mentioned, a further advantage of the third embodiment of the present invention lies in the reduction in energy consumption, by applying this embodiment, in fact, which provides for the complete elimination of the molten salt system and a direct supply of heat to the reactor by electric energy, all the heat lost is recovered both directly, i.e. which exits with the combustion fumes, and indirectly by dispersion into the environment from the hot surfaces of the molten salt circulation circuit. In this case the energy consumption is equal only to the requirement of the reactor which will therefore be 12,135,740 Kcal/h (1,013 Kg/h X 11,980 Kcal/h) with a decrease of 2,144,420 Kcal/h (14,280,160 - 12,135,740).

The first or second embodiment of the invention are also characterized by the complete elimination of the carbon dioxide discharge into the atmosphere, whereas the energy saving drops slightly to about 2,000,000 Kcal/h (the dispersions of the molten salt circuit are in fact still present, which, however, only account for about 1%).

| In short: | methane Kg/h | energy Kcal/h | CO₂ produced Kg/h |
|---|---|---|---|
| System according to the state of the art | 1192 | 14280160 | 3278 |
| 1st or 2nd embodiment of the invention | 1025 | 12279500 | 0000 |
| 3rd embodiment of the invention | 1013 | 12135740 | 0000 |

## Claims

1. A method for revamping a plant for the production of melamine which comprises the replacement of a heat supply system to the reactor already present in the plant with a heat supply system to the reactor suitable for using energy from renewable or partially renewable sources, said plant not producing any emission of CO₂ into the atmosphere following the supply of heat to the reactor.

2. The revamping method according to the previous claim 1, wherein the replacement of the heat supply system to the reactor already present in the plant comprises the following steps:
- the combustion furnace, suitable for supplying the necessary heat to the circulation system of molten salts, and relative accessory elements, is replaced by an electrically heated furnace, keeping the configuration of the reactor and the molten salt circulation system of the plant unchanged; or
- the combustion furnace, suitable for supplying the necessary heat to the circulation system of molten salts, and relative accessory elements, is replaced by a heat exchanger suitable for supplying the necessary heat to the circulation system of molten salts, connected to a heat recovery system, direct or indirect, from renewable or partially renewable sources, preferably powered by electric or solar energy, keeping the configuration of the reactor and the molten salt circulation system of the plant unchanged; or
- the combustion furnace, suitable for supplying the necessary heat to the circulation system of molten salts, the relative accessory elements, and the system suitable for circulating the molten salts through the reactor, consisting of a tank - pumps - internal and external pipes, are replaced by an electric heating system applied directly to the reactor.

3. A melamine production plant **characterized in that** the heat supply system to a melamine synthesis reactor is a supply system suitable for using energy from renewable or partially renewable sources which comprises:
- an electrically heated furnace suitable for supplying heat to a circulation system of molten salts and a circulation system of molten salts; or
- a heat exchanger suitable for supplying heat to a circulation system of molten salts, connected to a direct or indirect heat recovery system from renewable or partially renewable sources, preferably powered by electric or solar energy, and a circulation system of molten salts; or
- an electric heating system applied directly to the reactor,
said plant not producing any emission of CO₂ into the atmosphere following the supply of heat to the reactor.

4. The plant according to claim 3, wherein the heat supply system to the reactor is the electric heating system applied directly to the reactor wherein the internal process side of the reactor (1) comprises:
- a plurality of tubes (10) inside which electric heating elements (9) are housed, wherein the external side of the tubes (10) is in contact with the process fluids to which it supplies the necessary reaction heat;
- a plurality of heating elements (9) powered by electric energy (8), situated inside the tubes (10);
- a central internal circulation tube (6) suitable for guaranteeing the internal circulation of the process fluids;
- one or more inlets (F) of raw materials and one or more outlets (G) of the reaction products.

5. A process for the production of melamine operating at high pressure, i.e. without the use of a catalyst, **characterized in that**, in the synthesis step of melamine from urea, the heat to the reactor is supplied directly or indirectly through a renewable or partially renewable energy source, preferably electric or solar energy, more preferably electric energy.
